(19) Europäisches Patentamt
European Patent Office
Office européen des brevets

(11) EP 0 499 923 B1

(12) EUROPÄISCHE PATENTSCHRIFT

(45) Veröffentlichungstag und Bekanntmachung des Hinweises auf die Patenterteilung:
**24.01.1996 Patentblatt 1996/04**

(51) Int Cl.$^6$: **C08F 220/36**, A61K 6/00

(21) Anmeldenummer: **92102153.1**

(22) Anmeldetag: **10.02.1992**

(54) **Dentaladhäsive**

Dental adhesive

Adhésif dentaire

(84) Benannte Vertragsstaaten:
**AT BE CH DE DK ES FR GB GR IT LI LU NL PT SE**

(30) Priorität: **22.02.1991 DE 4105550**

(43) Veröffentlichungstag der Anmeldung:
**26.08.1992 Patentblatt 1992/35**

(73) Patentinhaber: **BAYER AG**
**D-51368 Leverkusen (DE)**

(72) Erfinder:
- **Müller, Michael, Dr.**
  **W-5060 Bergisch Gladbach 2 (DE)**
- **Finger, Werner, Prof. Dr.**
  **W-4040 Neuss 21 (DE)**
- **Podszun, Wolfgang, Dr.**
  **W-5000 Köln 80 (DE)**
- **Winkel, Jens, Dr.**
  **W-5000 Köln 71 (DE)**

(56) Entgegenhaltungen:
**EP-A- 0 234 934**
**EP-A- 0 355 562**
**EP-A- 0 394 787**
**DE-A- 2 739 282**
**FR-A- 2 301 539**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 499 923 B1

**Beschreibung**

Die Erfindung betrifft Zubereitungen zur Verwendung als Adhäsivkomponente zur Behandlung der Zahnhartsubstanz, ein Verfahren zu ihrer Herstellung und ihre Verwendung.

Die Zahnhartsubstanz ist aus Zahnschmelz und Zahnbein (Dentin) aufgebaut, die sich in ihrer Zusammensetzung stark unterscheiden. Zahnschmelz ist weitgehend mineralisch, insbesondere aus Calciumhydroxylapatit aufgebaut. Dentin besteht hingegen zu einem erheblichen Anteil aus organischen Bausteinen wie Collagen und anderen Proteinen und ist stärker wasserhaltig.

Im Rahmen der vorliegenden Erfindung werden die Adhäsivkomponenten bevorzugt zur Behandlung von Zahnschmelz und Dentin im Zusammenhang mit Zahnreparaturen verwendet.

Besonders im Dentalbereich werden härtende, polymere Materialien als Füllmaterialien bei Zahnreparaturen verwendet. Als härtende, polymere Materialien werden im allgemeinen Füllungen auf Acrylatbasis bevorzugt. Diese polymeren Füllungen haben jedoch den Nachteil, daß sie schlecht an der Zahnhartsubstanz haften bleiben. Um dieses Problem zu lösen, hat man bisher teilweise Unterschneidungen am Zahnbein vorgenommen; dazu war es erforderlich, über den angegriffenen Bereich hinaus, beachtliche Mengen an gesundem Dentin zu entfernen.

Nach einer anderen Methode äzt man das Dentin und die Schmelzoberfläche mit Säuren, wie z.B. Phosphorsäure, an, und nimmt dann die Füllung vor. Abgesehen davon, daß die Säure eine Reizwirkung im Mundbereich ausübt, dringt sie auch leicht durch die Dentinkanäle in den Zahn und reizt den Nerv.

Geeignete Grundierungsmittel, die vor dem Aufbringen des Füllungsmaterials auf den geätzten Schmelz aufgetragen werden, sind gängige difunktionelle Methacrylate. Diese viskosen Öle, insbesondere 2,2-Bis-[4'(3"-methacryloyl-2"-hydroxypropoxy)phenyl]-propan (Bis-GMA), Triethylenglykoldimethacrylat oder einfache Alkandioldimethacrylate bewirken zwar eine Verbundhaftung zum Schmelz, jedoch nicht zum Dentin. Hierfür wurden spezielle Dentin-Haftvermittler entwickelt. Sie verbessern die Verbundhaftung zum Dentin, sind aus klinischer Sicht aber nur unzureichend wirksam, da sich Randspalte zwischen Dentin und Füllungsmaterial ausbilden, die die Ursache für Sekundärkaries und Verfärbungen sind.

In der DE-OS 38 28 169 wird eines der am besten geeigneten Dentinadhäsive beschrieben. Die wirksamen Zubereitungen enthalten

A) Formamidgruppen enthaltende (Meth)acrylsäureester der Formel

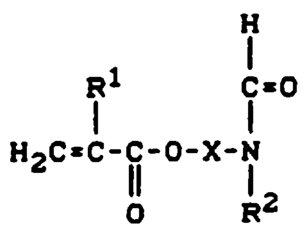

(I),

in der

$R^1$ Wasserstoff oder Methyl bedeutet,

$R^2$ Wasserstoff, gegebenenfalls durch Hydroxy, Carboxy, Halogen oder Amino der Formel

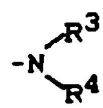

in der

$R^3$ und $R^4$ gleich oder verschieden sind und Wasserstoff oder Niederalkyl bedeuten,
substituiertes Alkyl ($C_1$ bis $C_{12}$), Aryl ($C_6$ bis $C_{12}$) oder Aralkyl ($C_7$ bis $C_{14}$) bedeutet, und

X ein zweiwertiger, gegebenenfalls durch Hydroxy, Carboxy, Halogen oder Amino der Formel

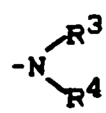

in der

$R^3$ und $R^4$ die obengenannte Bedeutung haben,
substituierter aliphatischer ($C_1$ bis $C_{24}$) und/oder cycloaliphatischer ($C_5$ bis $C_8$) und/oder aromatischer ($C_6$ bis $C_{12}$) Rest, der gegebenenfalls eine oder mehrere Sauerstoff-, Schwefel- und/oder $-NR^3$-Brücken enthalten kann,
wobei

$R^3$ die obengenannte Bedeutung hat,

bedeutet,
und gegebenenfalls

B) Initiatoren, gegebenenfalls unter Zusatz von Coaktivatoren, C) Lösungsmittel,

D) Carbonylverbindungen,

E) Füllstoffe,

F) (Meth)acrylsäureester, die Vernetzungen ausbilden können.

In der Tat ist es nicht möglich aus den in der DE-OS 38 28 169 beschriebenen Komponenten A) bis F) eine Anmischung herzustellen, die eine ausreichende Verbundhaftung sowohl zum Dentin als auch zum Zahnschmelz gewährleistet.

Der vorliegenden Erfindung lag die Aufgabe zugrunde diesen Mangel zu beheben. Hierzu war es nicht nur notwendig, innerhalb der weiten angegebenen Konzentrationsgrenzen für die Komponenten A) bis F) der DE-OS 38 28 169 Vorzugsbereiche zu ermitteln, sondern zur Bewältigung der erfindungsgemäßen Aufgabe mußte eine weitere Komponente ergänzt werden. Überraschenderweise ließ sich eine ausreichende Verbundhaftung zu Dentin und Zahnschmelz erst nach Zugabe einer Säure erreichen. Dies war insbesondere deshalb nicht voraussehbar, da Säuren das Dentin bekanntermaßen durch Demineralisierung schwächen und dadurch die Verbundhaftung zum Dentin erniedrigen.

Wesentliche Vorteils der erfindungsgemäßen Adhäsive sind

- Schmelz und Dentin werden in einem Arbeitsgang mit demselben Adhäsiv versehen.

- Die Bindungsfestigkeit ist im Vergleich zu bisher bekannt gewordenen derartigen Adhäsiven deutlich höher.

- Die physiologische Verträglichkeit ist im Vergleich zu bisher bekannten Adhäsiven, insbesondere aldehydhaltigen Systemen, erheblich besser.

- Überraschenderweise kann durch die Anwendung erfindungsgemäßer Adhäsive erstmals die Bildung von Randspalten völlig verhindert werden. So kann z.B. bei der Zubereitung gemäß Beispiel 1 eine 100 %ige Randspaltfreiheit festgestellt werden, wohingegen bei der Zubereitung gemäß Vergleichsbeispiel 5 in 40 X der Fälle ein Randspalt auftritt.

Das erfindungsgemäße Adhäsiv zur Behandlung der gesamten Zahnhartsubstanz enthält

a) Formamidgruppen enthaltende (Meth)acrylsäureester der Formel

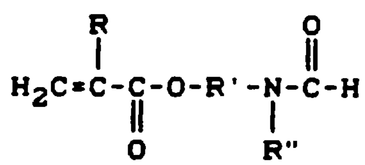

(1)

in der

R Wasserstoff oder Methyl bedeutet,

R' ein zweiwertiger aliphatischer ($C_2$ bis $C_5$) Rest bedeutet und

R" Wasserstoff oder ein einwertiger Alkylrest ($C_1$ bis $C_3$) bedeutet,

b) (Meth)acrylsäureester, die Vernetzungen ausbilden können,

c) Lösungsmittel,

d) Säuren und
gegebenenfalls

e) Initiatoren, Coaktivatoren, Füllstoffe und Dispergatoren

und gegebenenfalls weitere übliche Zusätze.

(Meth)-acrylsäureester im Rahmen der vorliegenden Erfindung sind die Ester der Acrylsäure und der Meth-acrylsäure.

Die Substituenten der Formamidgruppen enthaltenden (Meth)-acrylsäureester (a) im Rahmen der allgemeinen Formel (1) haben im allgemeinen die folgende Bedeutung:

Ein zweiwertiger aliphatischer $C_2$- bis $C_5$-Rest steht für Ethylen, Propylen, Butylen, Pentylen, 2,2-Dimethylpropylen oder 1- bzw. 2-Methylbutylen. Bevorzugt wird Propylen oder Butylen, besonders bevorzugt ist Ethylen.

Der einwertige Rest R" steht für Wasserstoff oder einen $C_1$-, $C_2$- oder $C_3$-Rest, worunter Methyl-, Ethyl-, n-Propyl- oder Isopropylreste zu verstehen sind. Bevorzugt ist Wasserstoff oder Ethyl. Methyl ist besonders bevorzugt.

Beispielsweise seien die folgenden Formamidgruppen enthaltenden (Meth)-acrylsäureester genannt:

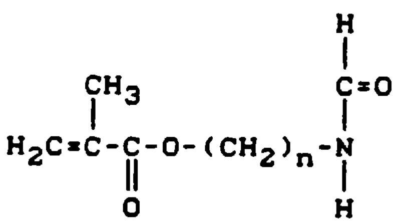

mit n = 2 - 5 (1a)

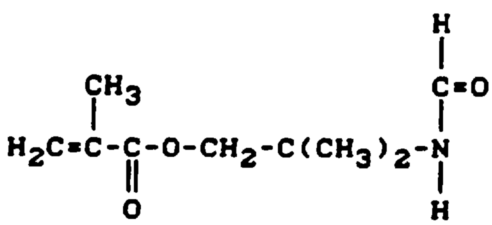

(1b)

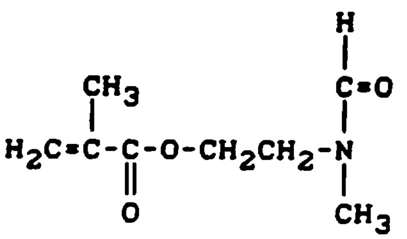

(1c)

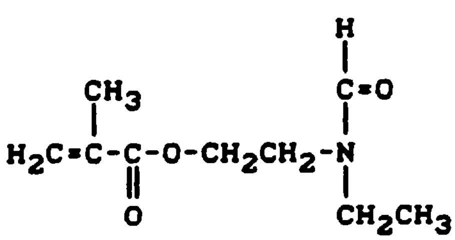

(1d)

Besonders bevorzugt ist N-Methacryloyloxyethyl-N-methylformamid (1c).

Die Herstellung der Formamidgruppen enthaltenden (Meth)-acrylsäureester ist an sich bekannt (DE-A-1 770 964 und DE-A-2 507 189). Beispielsweise können die Formamidgruppen enthaltenden (Meth)-acrylsäureester durch Umsetzung von Alkanolaminen mit Ameisensäureestern und (Meth)-acrylsäurechlorid hergestellt werden.

Die erfindungsgemäßen Zubereitungen enthalten im allgemeinen 2 bis 80 Gew.-Teile, bevorzugt 5 bis 50 Gew.-Teile und besonders bevorzugt 14 bis 25 Gew.-Teile der Formamidgruppen enthaltenden (Meth)acrylsäureester.

Die erfindungsgemäßen Zusammensetzungen können (Meth)-acrylsäureester enthalten, die Vernetzungen ausbilden können. (Meth)-acrylsäureester, die Vernetzungen ausbilden können, enthalten im allgemeinen 2 oder mehr polymerisierbare aktive Gruppen im Molekül. Bevorzugt seien Ester der (Meth)acrylsäure mit 2- bis 5-wertigen Alkoholen mit 2 bis 30 Kohlenstoffatomen genannt. Besonders bevorzugt werden Alkoxy(meth)acrylate und Urethangruppen enthaltende (Meth)acrylate.

Beispielsweise seien (Meth)-acrylsäureester der Formel

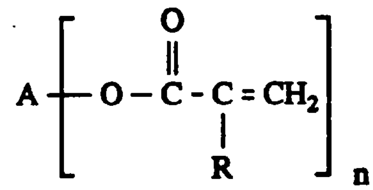

in der

A einen geradkettigen, verzweigten, cyclischen, aliphatischen, aromatischen oder gemischt aliphatisch-aromatischen Rest mit 2 bis 25 C-Atomen, der durch -O- oder NH-Brücken unterbrochen und mit Hydroxy, Oxy, Carboxy, Amino oder Halogen substituiert sein kann,
bedeutet,

R H oder Methyl bedeutet und

n für eine ganze Zahl von 2 bis 8, vorzugsweise 2 bis 4, steht,
genannt.

Vorzugsweise seien Verbindungen der folgenden Formeln genannt:

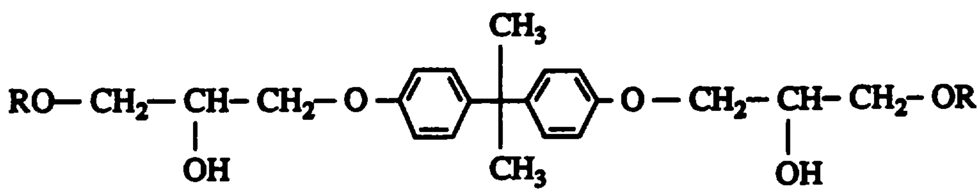

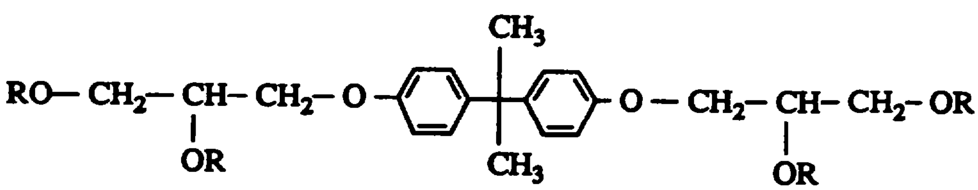

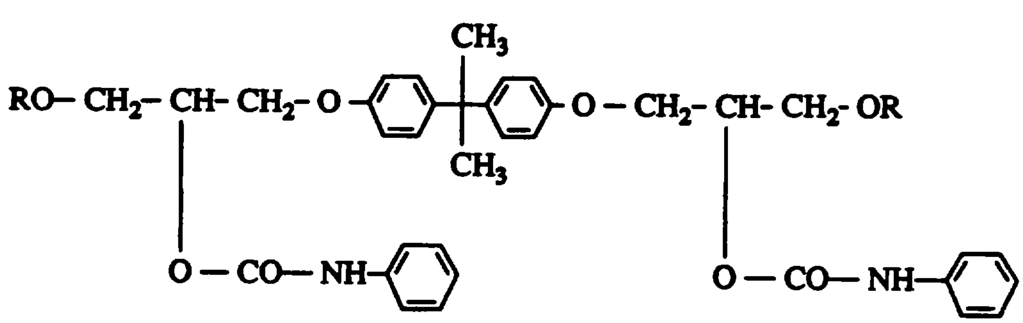

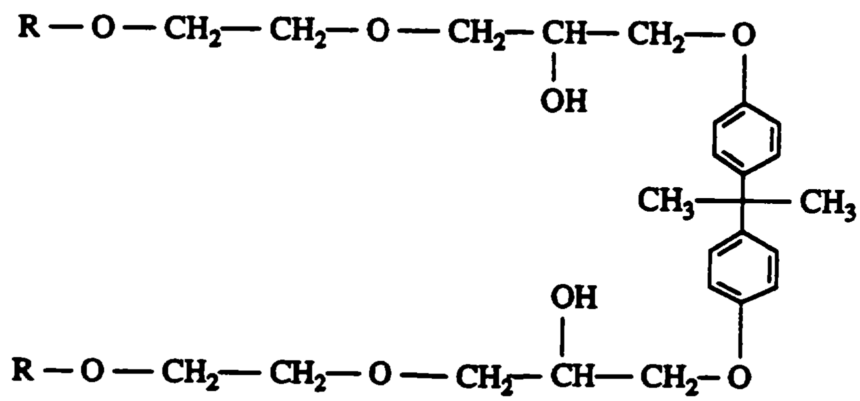

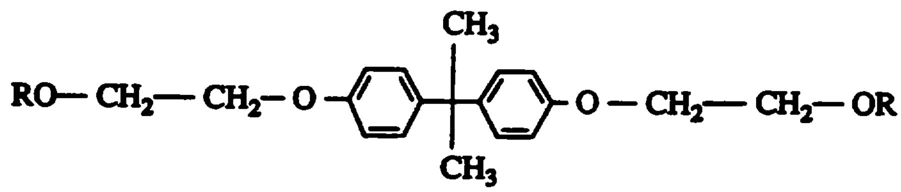

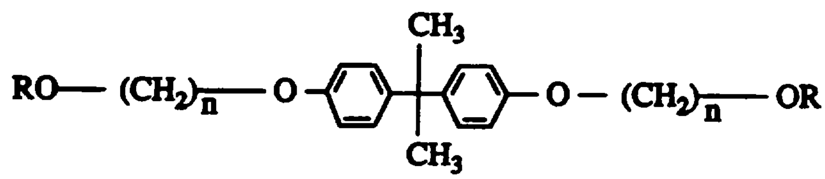

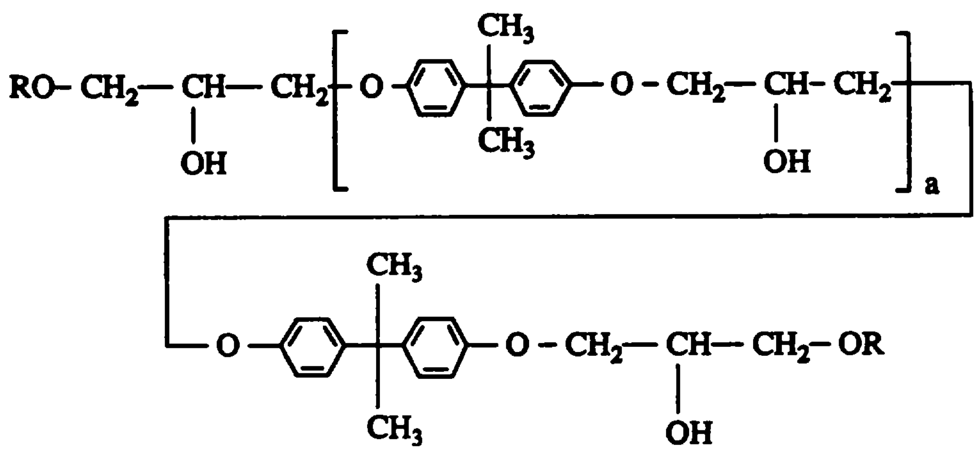

wobei a eine Zahl von 1 bis 4 bedeutet

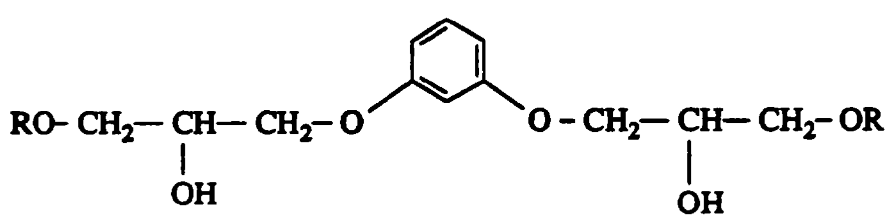

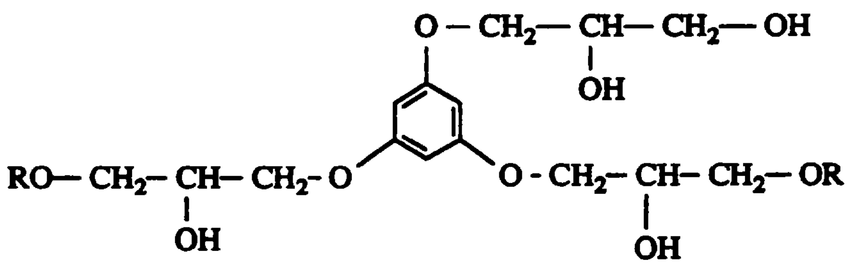

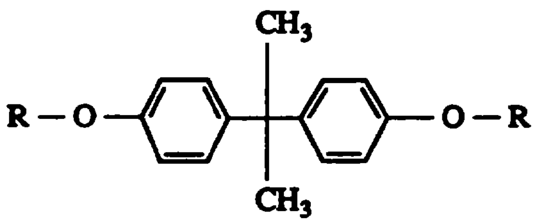

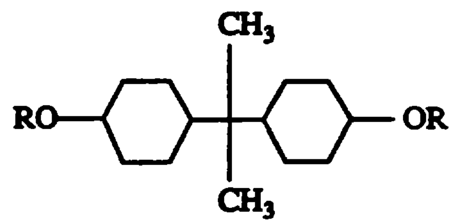

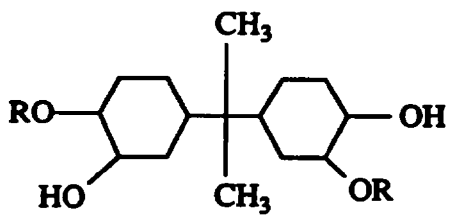

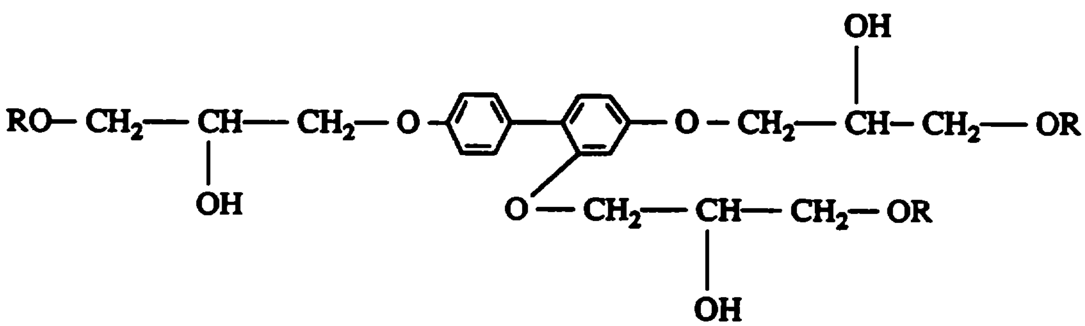

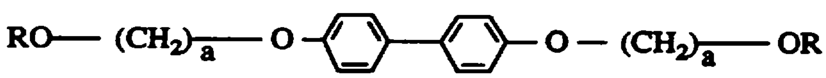

wobei a eine Zahl von 1 bis 4 bedeutet,

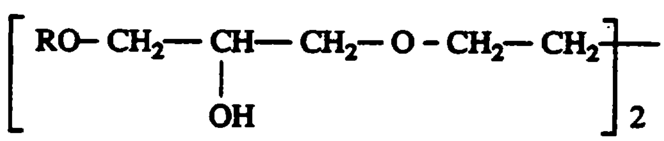

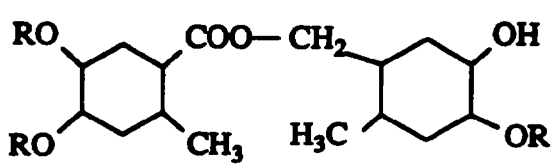

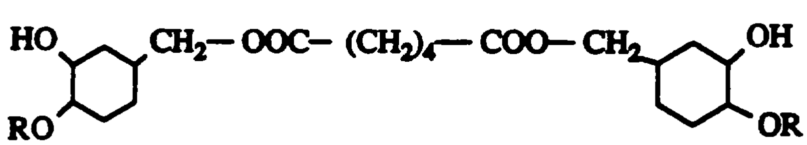

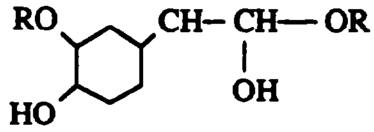
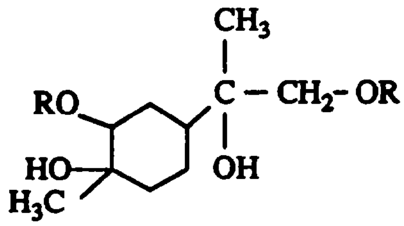
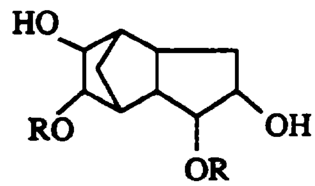

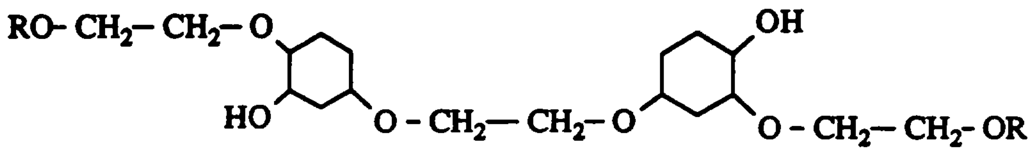

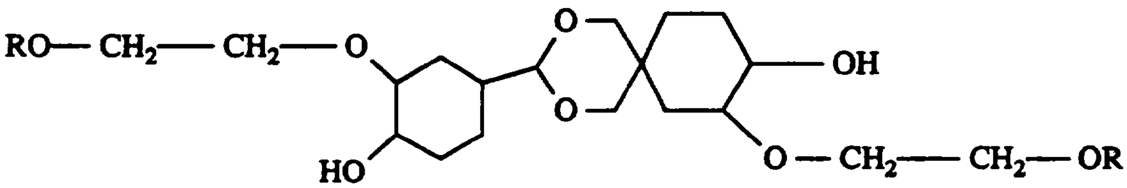

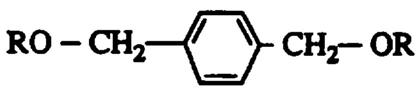
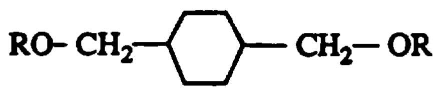

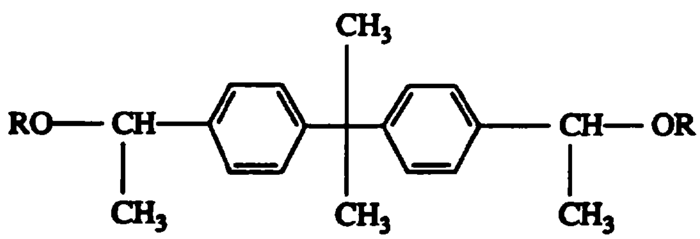

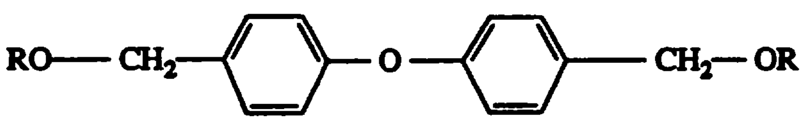

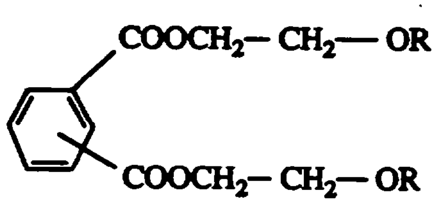
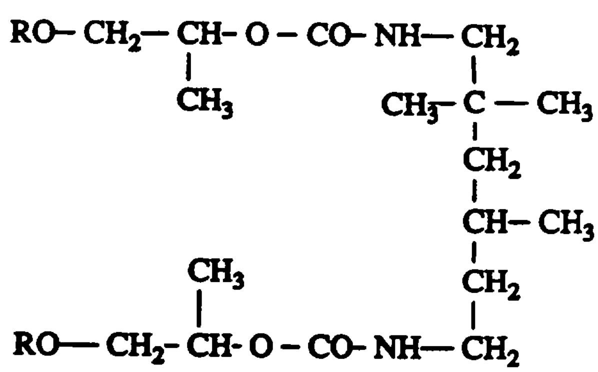

in der ortho-, meta- oder para-Form

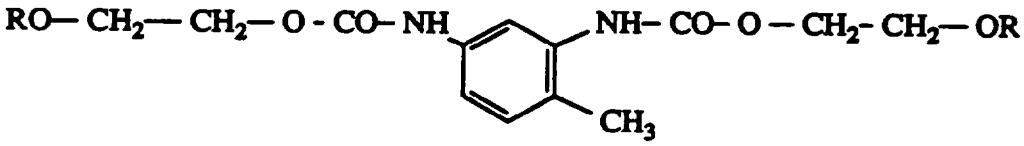

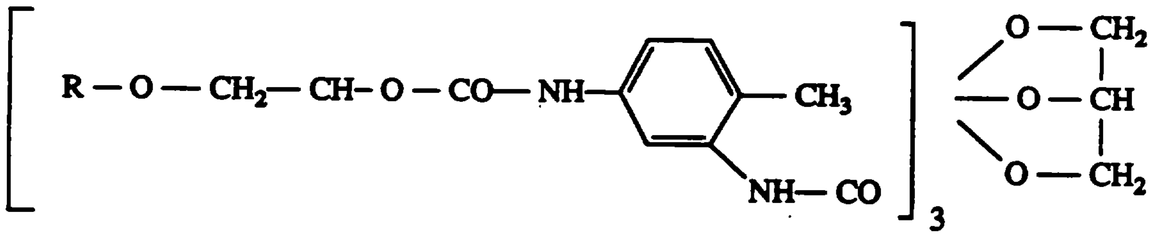

worin R für

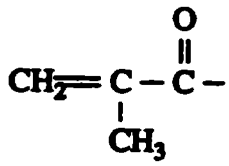

oder

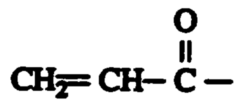

steht.

Außerdem seien Derivate des Tricyclodecans (EP-A 0 023 686) und Umsetzungs produkte aus Polyolen, Diisocyanaten und Hydroxyalkylmethacrylaten (DE-A-37 03 120, DE-A 37 03 080 und DE-A 37 03 130) genannt. Beispielsweise seien die folgenden Monomeren genannt:

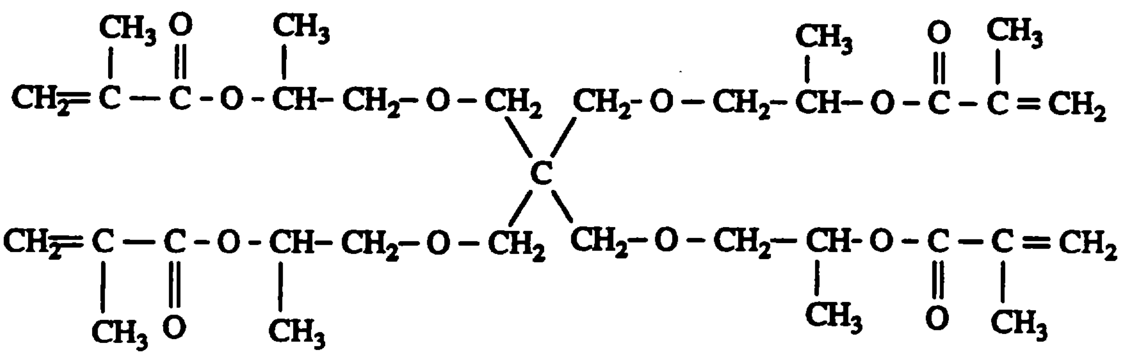

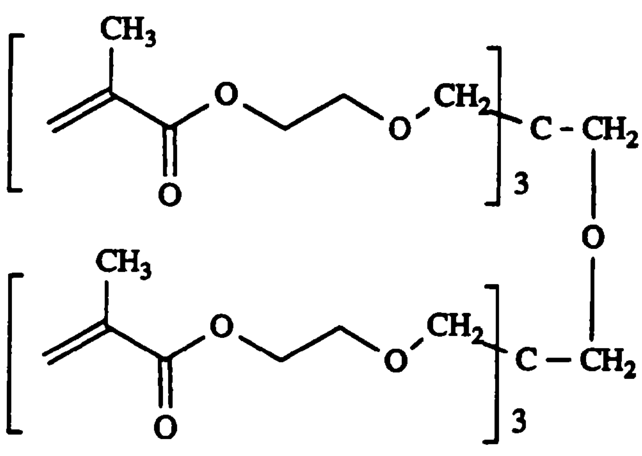

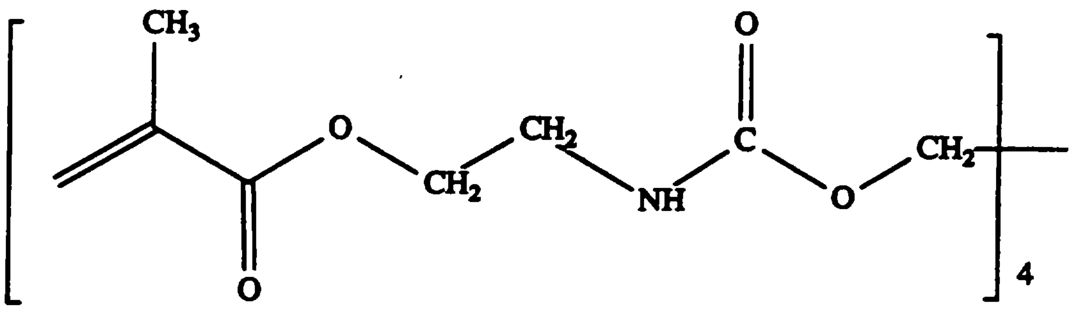

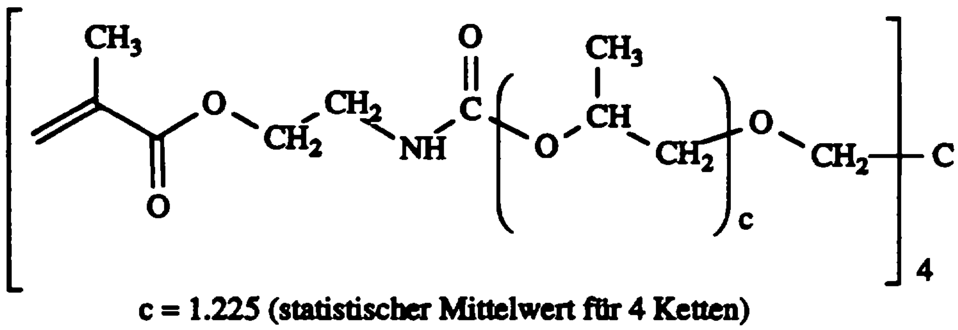

c = 1.225 (statistischer Mittelwert für 4 Ketten)

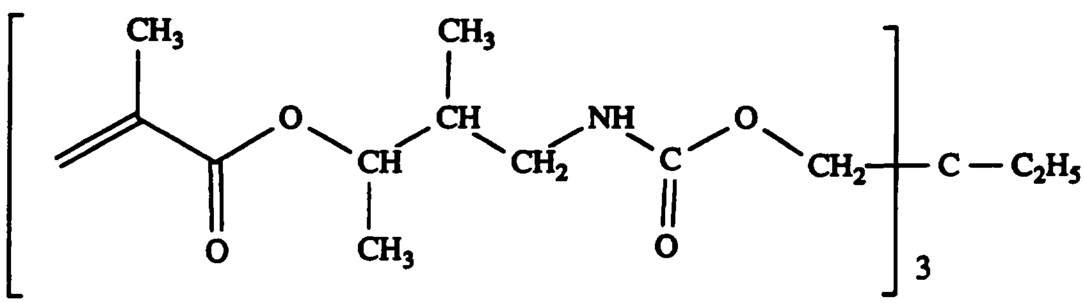

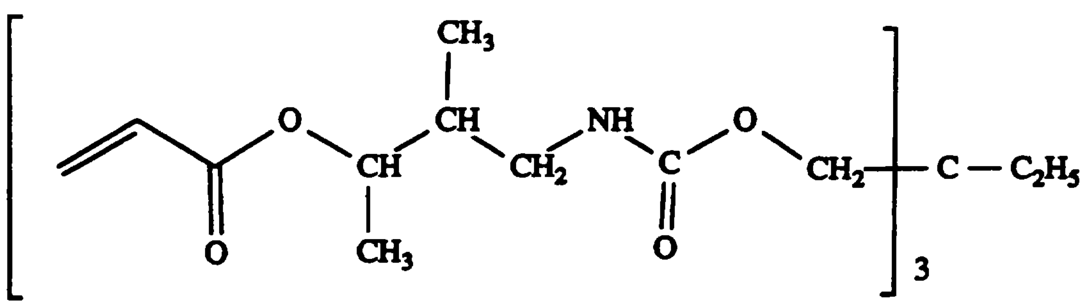

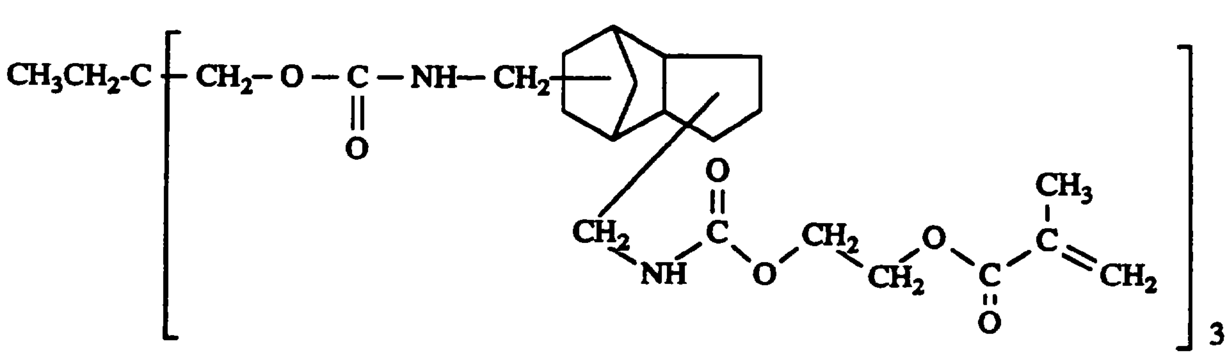

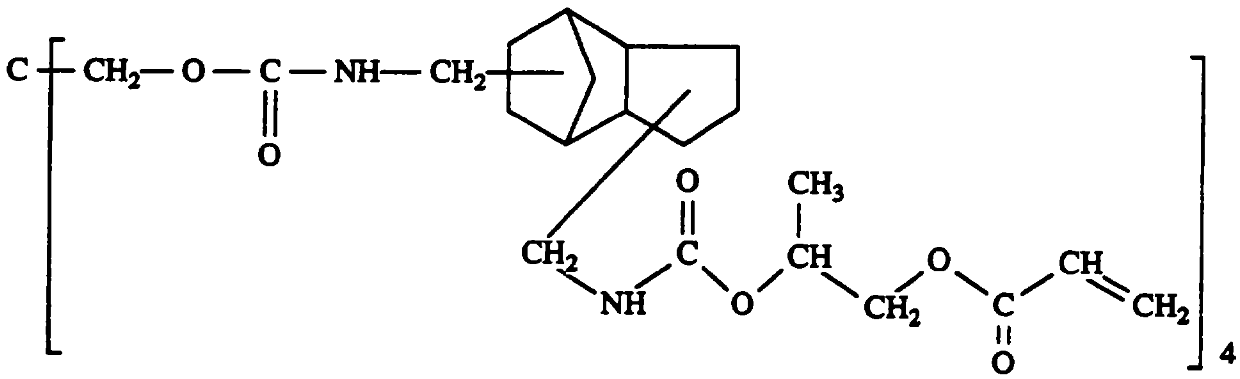

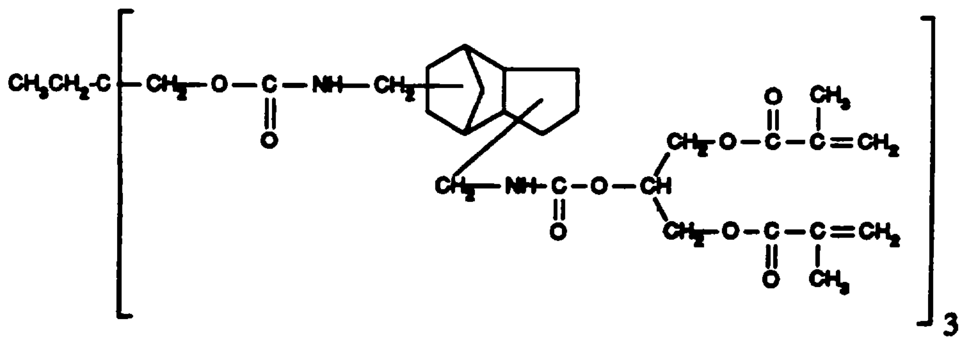

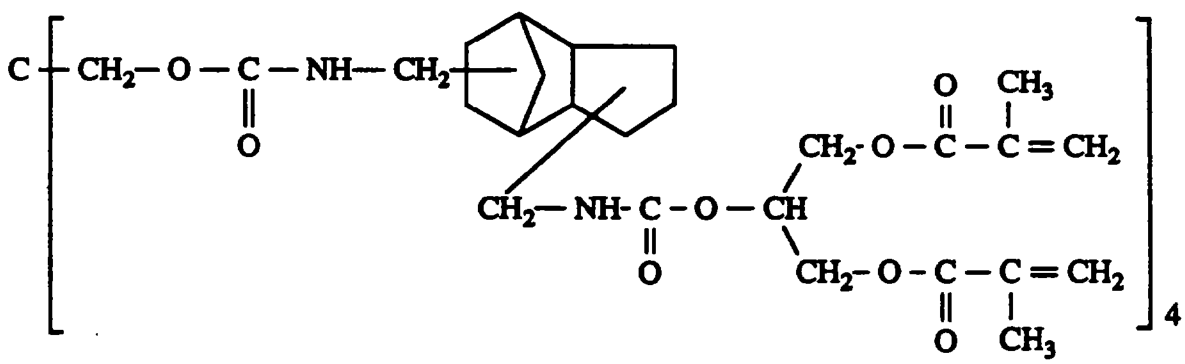

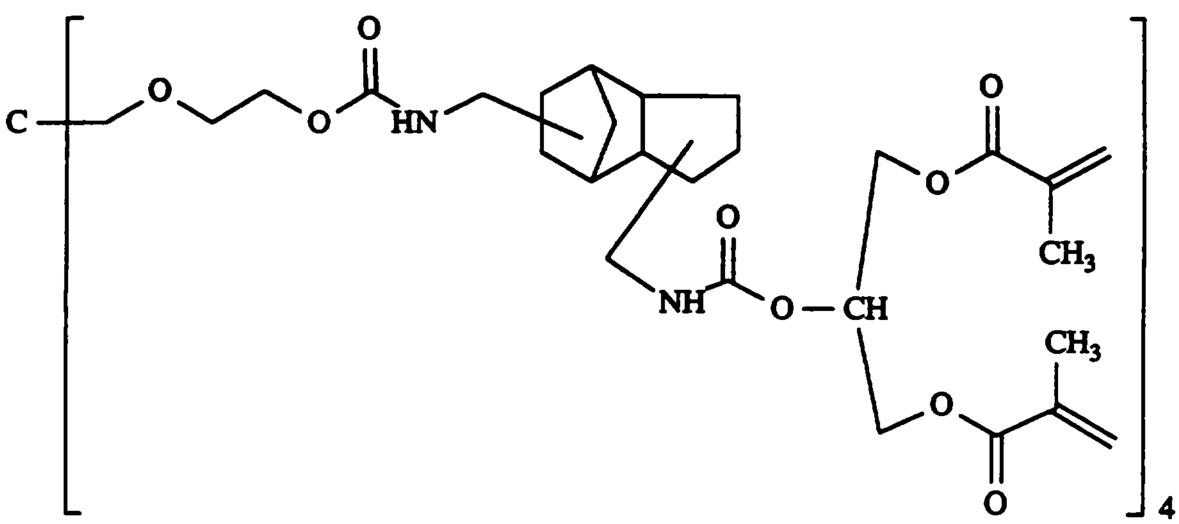

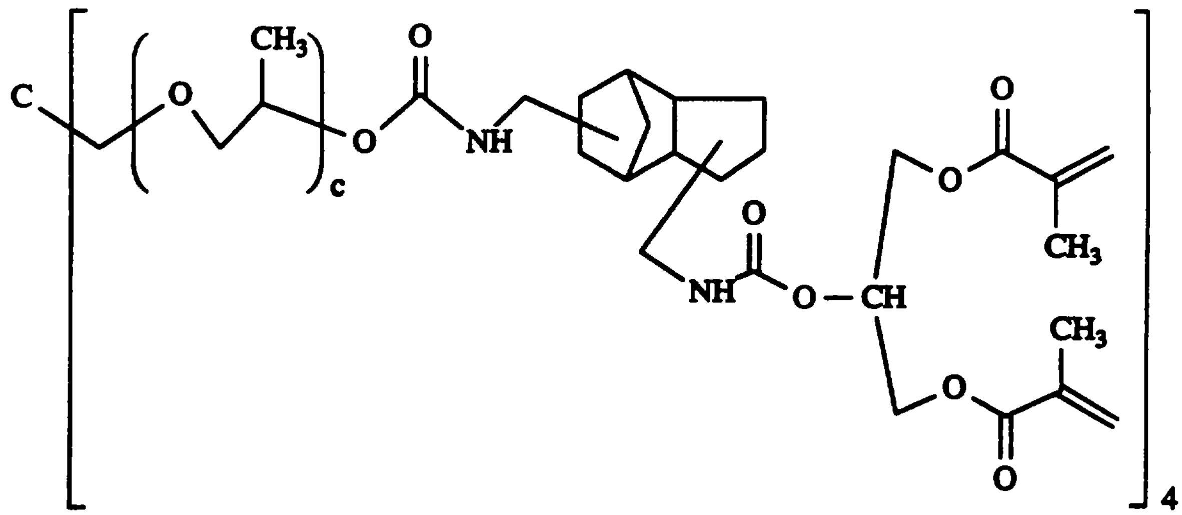

c = 1.225 (statistischer Mittelwert für 4 Ketten)

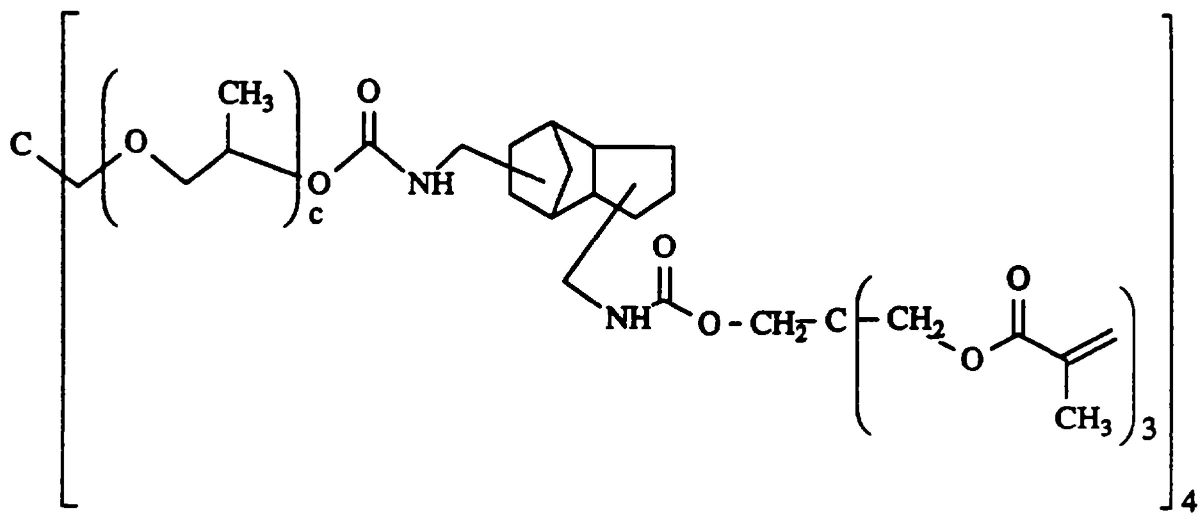

c = 1.225 (statistischer Mittelwert für 4 Ketten)

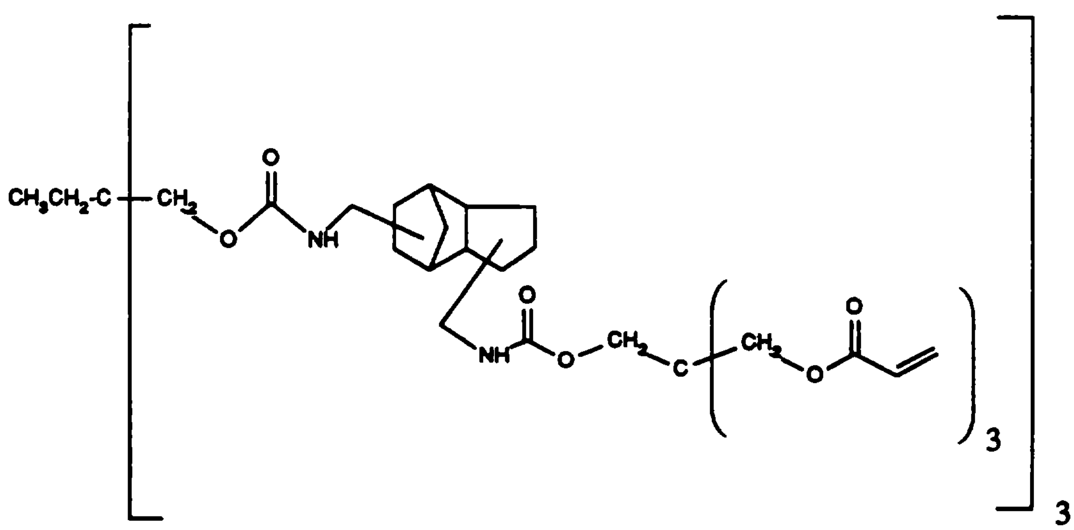

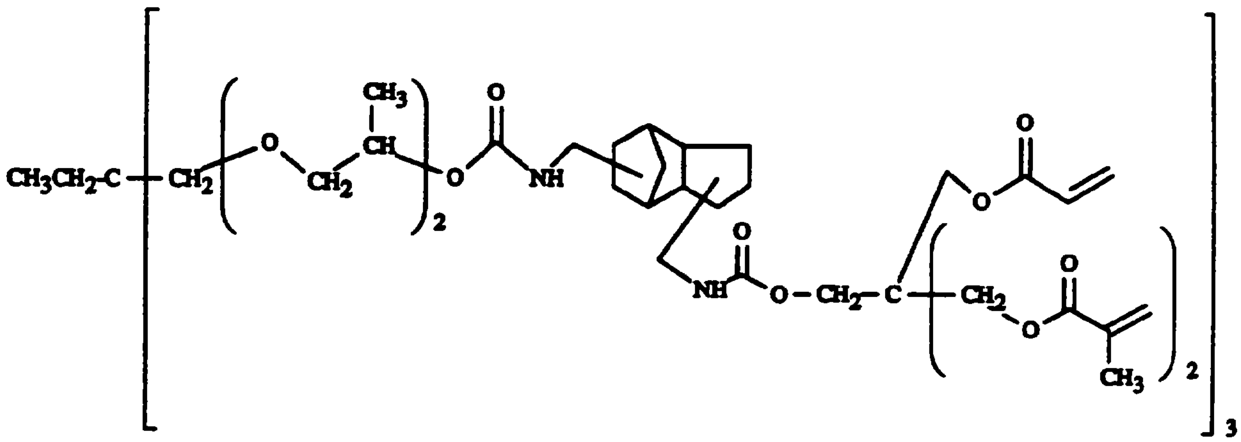

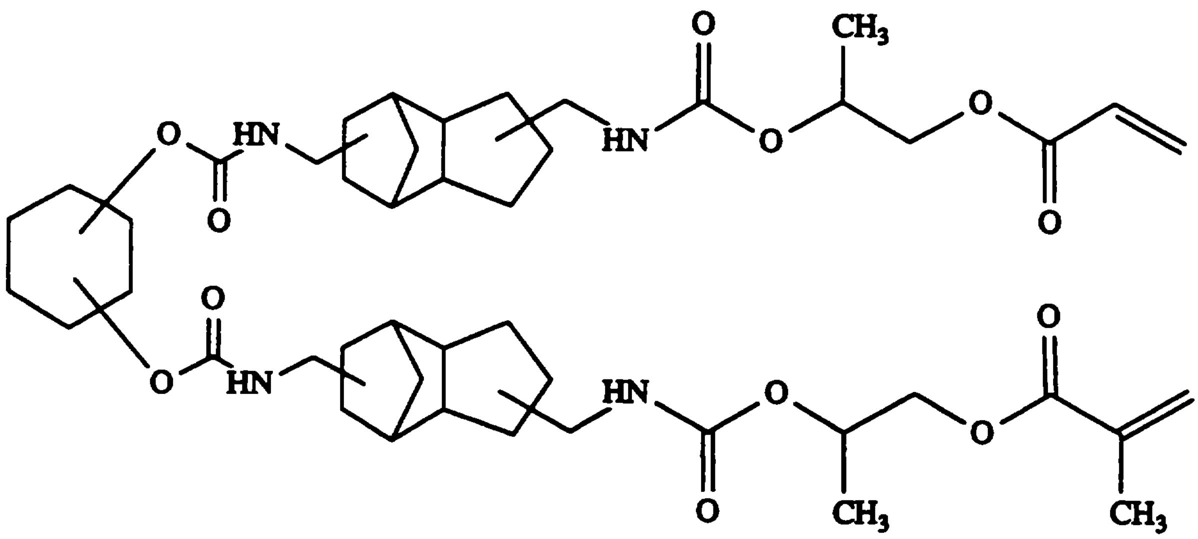

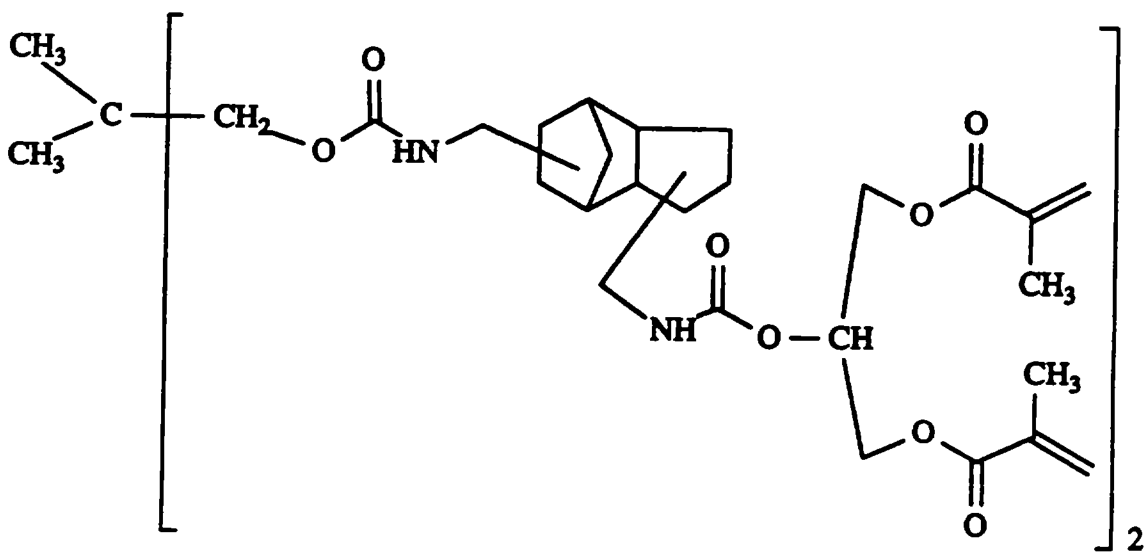

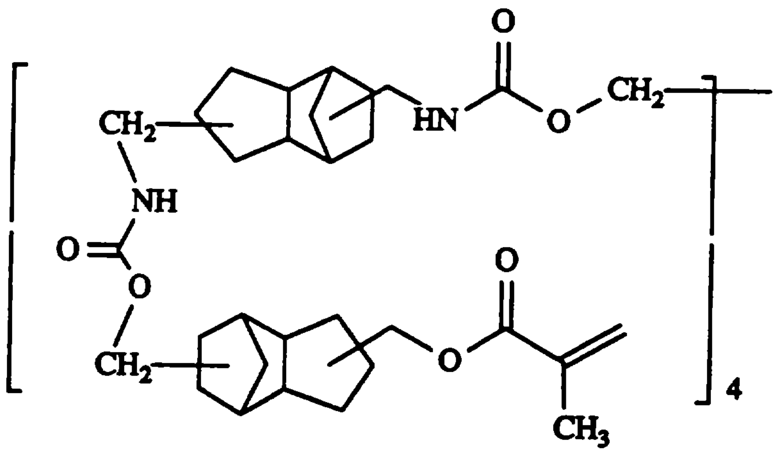

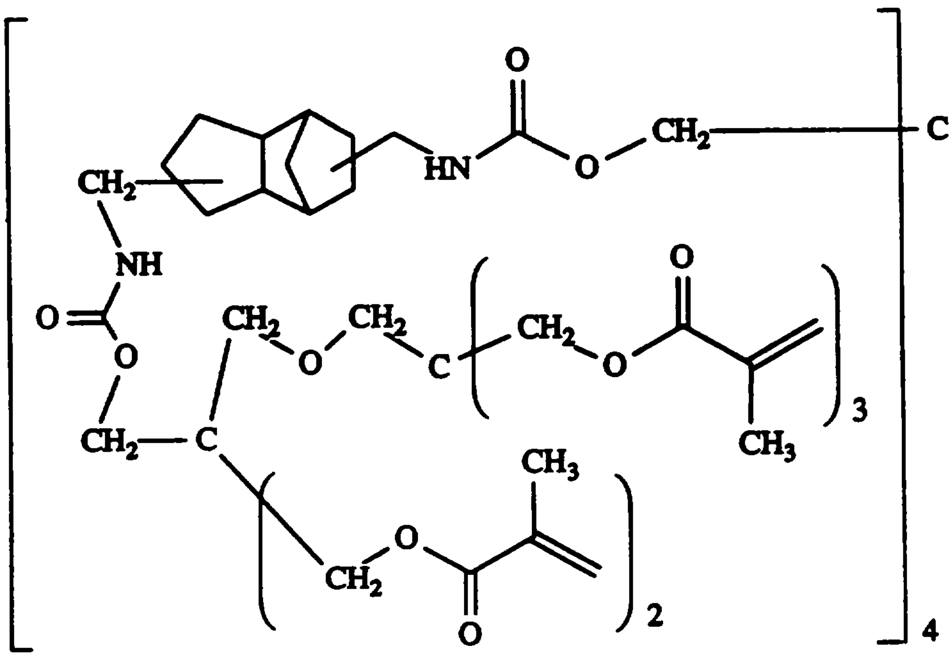

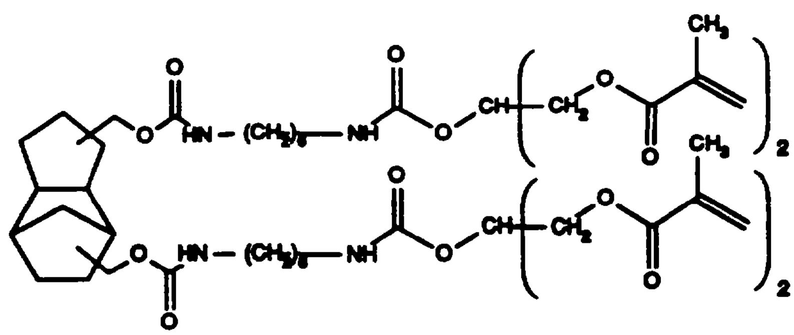

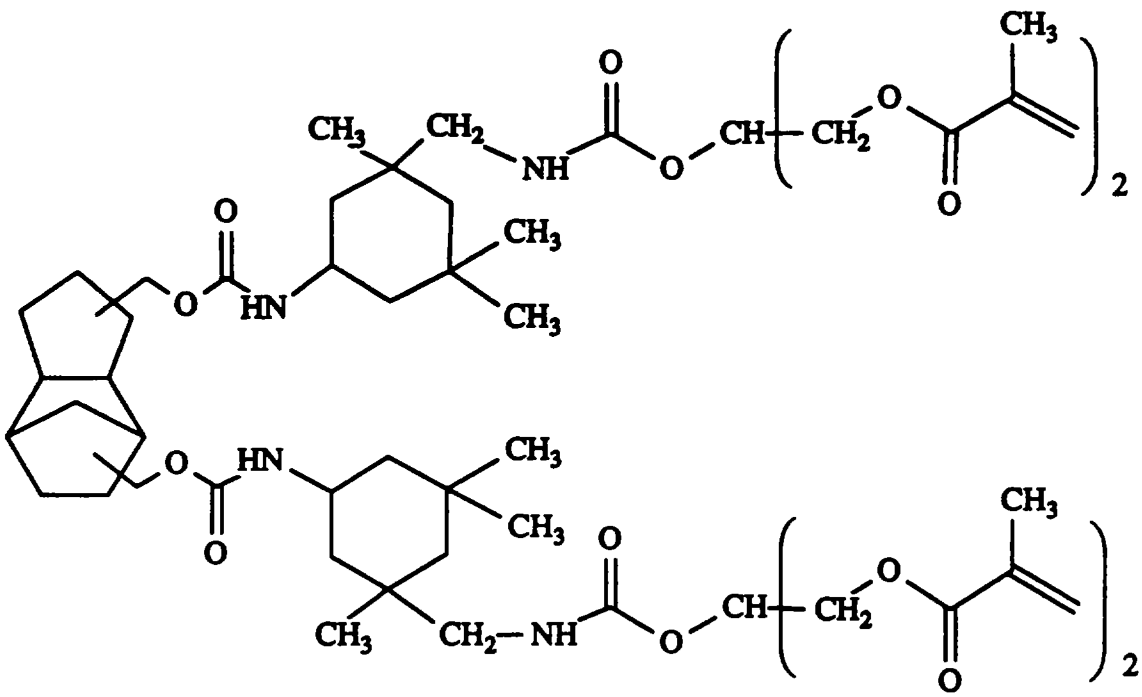

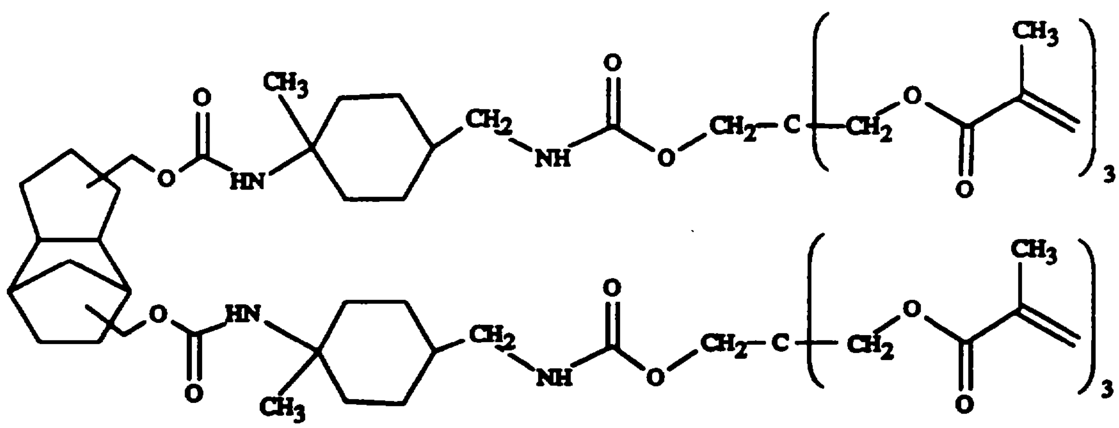

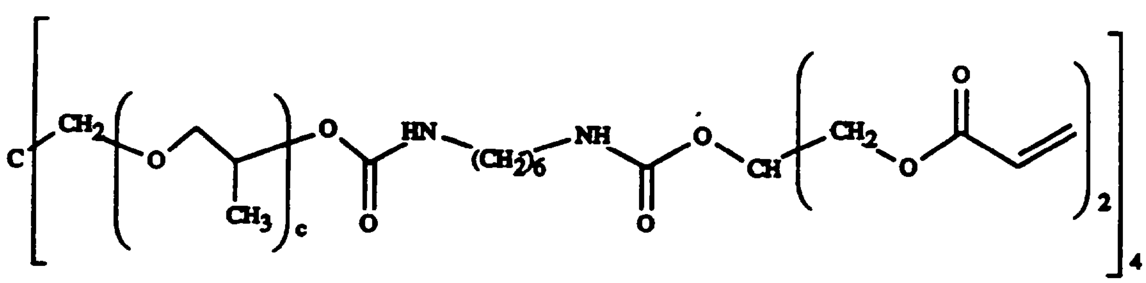

c = 1.225 (statistischer Mittelwert für 4 Ketten)

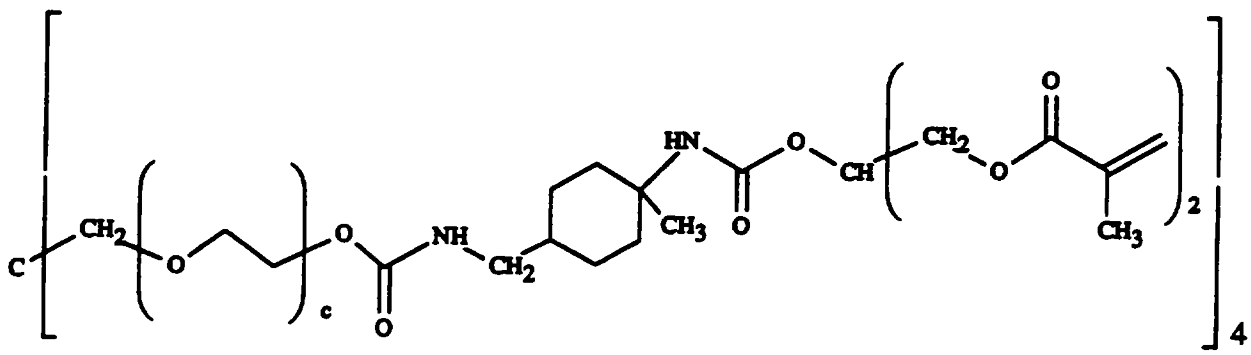

c = 1.225 (statistischer Mittelwert für 4 Ketten)

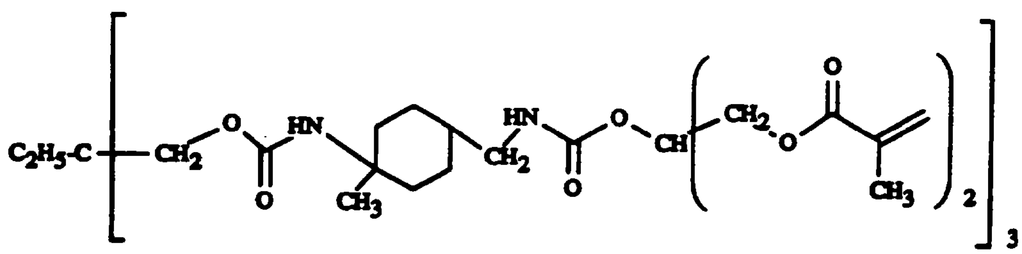

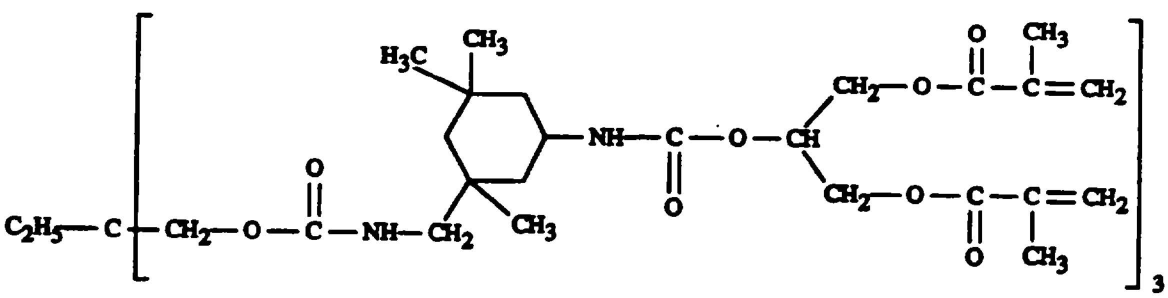

Als Methacrylsäureester besonders bevorzugt wird das sogenannte Bis-GMA der Formel

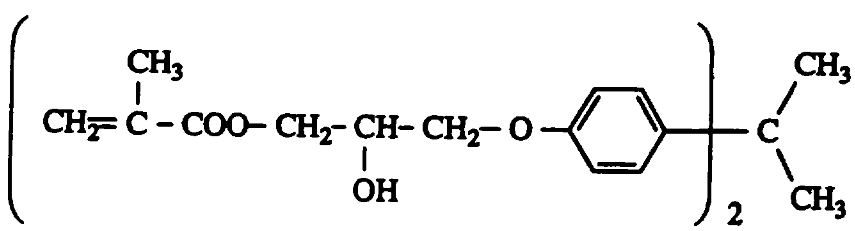

Selbstverständlich ist es möglich, Mischungen der verschiedenen (Meth)-acrylsäureester, die Vernetzungen ausbilden können, einzusetzen. Beispielsweise seien Mischungen von 20 bis 70 Gew.Teilen Bis-GMA und 30 bis 80 Gew.-Teilen Triethylenglykoldimethacrylat genannt.

Die erfindungsgemäßen Zubereitungen enthalten im allgemeinen 0,3 bis 80 Gew.-Teile, bevorzugt 1 bis 50 Gew.-Teile und besonders bevorzugt 4 bis 30 Gew.-Teile der Methacrylsäureester, die Vernetzungen ausbilden können.

Die Lösungsmittel im Rahmen der vorliegenden Erfindung sollen die Komponente lösen und sollen, durch die Anwendung bedingt, untoxisch sein. Bevorzugt seien Wasser und flüchtige organische Lösungsmittel wie Methanol, Ethanol, Propanol, Isopropanol, Aceton, Methylethylketon, Essigsäuremethyl- oder -ethylester bzw. Tetrahydrofuran genannt.

Im allgemeinen setzt man 10 bis 1000 Gew.-Teile, bevorzugt 30 bis 300 Gew.-Teile, besonders bevorzugt 50 bis 80 Gew.-Teile des Lösungsmittels, bezogen auf die gesamte Anmischung ein.

Als Säuren im Rahmen der vorliegenden Erfindung können Propion-, Malein-, Oxal-, Zitronen-, Wein-, Äpfel-, Brenztrauben- oder p-Toluolsulfonsäure verwendet werden. Bevorzugt ist Ameisensäure, besonders bevorzugt Essigsäure.

Man setzt im allgemeinen 0,3 bis 20, bevorzugt 1 bis 15 und besonders bevorzugt 2 bis 7 Gew.-Teile der Säure

bezüglich der gesamten Anmischung ein.

Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslösen.

Die sogenannten Fotopolymerisationsinitiatoren sind an sich bekannt (Houben-Weyl, Methoden der organischen Chemie, Band E20, Seite 80 ff, Georg Thieme Verlag Stuttgart 1987). Vorzugsweise handelt es sich um Monooder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketo-Derivate des Norbornans und substituierter Norbornane, Metallcarbonyle wie Pentacarbonylmangan oder Chinone wie 9,10-Phenanthrenchinon und Naphthochinon. Besonders bevorzugt ist Campherchinon.

Die erfindungsgemäßen Zubereitungen enthalten im allgemeinen 0 bis 2 Gew.-Teile, bevorzugt 0,1 bis 0,5 Gew.-Teile des Initiators, bezogen auf 1 Gew.-Teil des Formamidgruppen enthaltenden (Meth)-acrylsäureesters. Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N',N'-Tetraalkylalkylendiamine, Barbitursäure und Dialkylbarbitursäuren.

Die Coaktivatoren werden im allgemeinen in einer Menge von 0 bis 4 Gew.-%, bevorzugt 0,2 bis 1 Gew.-%, bezogen auf die Menge an polymerisierbaren Verbindungen eingesetzt.

Als weitere Komponente können die erfindungsgemäßen Zusammensetzungen Füllstoffe enthalten. Als Füllstoffe werden feine Pulver bevorzugt, die einen Teilchendurchmesser im Bereich von 0,1 bis 100 µm (gegebenenfalls auch in einer polydispersen Verteilung) haben. Füllstoffe können im Dentalbereich übliche Füllstoffe (R. S. Baratz, J. Biomat. Applications, Vol 1, 1987, S 316 ff) wie anorganische Gläser, Siliziumdioxid-, Aluminiumoxidoder Quarzpulver sein.

Durch einen Anteil an Füllstoffen in den erfindungsgemäßen Zubereitungen entstehen Klebezemente, die besonders zur Befestigung von Brücken-, Kronen- und anderen verblendmaterialen geeignet sind.

Der Anteil des Füllstoffs beträgt im allgemeinen 0 bis 80 Gew.-Teile, bevorzugt 40 bis 70 Gew.- Teile, bezogen auf die gesamte Zubereitung.

Erfindungsgemäß einsetzbare Dispergatoren können ionische und nichtionische Tenside sein.

Bevorzugte anionische Tenside sind beispielsweise: Phosphate wie Di-(2-ethylhexyl)-phosphat-Na-Salz, Alkoholsulfate wie Oleylalkoholsulfat, Alkyl($C_6$ bis $C_{20}$)benzolsulfonate und Alkyl($C_8$ bis $C_{24}$)sulfonate, insbesondere Sulfobernsteinsäuredioctylester-Na-Salz.

Bevorzugte kationische Tenside können beispielsweise sein: quartäre Ammoniumsalze wie beispielsweise Methyltrioctylammoniumchlorid und Methyl-tricaprylylammoniumchlorid.

Erfindungsgemäß einsetzbar sind auch nichtionogene Tenside in Form von Fettsäurederivaten von Polyolen oder des Ethylenoxids, ethoxylierte Fettalkohole und Phenole sowie amphotere Tenside wie Alkylaminoethansulfonsäuren. Besonders bevorzugt sind oberflächenaktive, hochmolekulare Verbindungen. Genannt seien hier wasserlösliche Polyvinylverbindungen wie Polyvinylacetat, Polymethacrylsäure und Polyacrylsäure sowie deren Alkalisalze, Copolymerisate aus Natriummethacrylat und Methacrylsäurealkylester. Weiterhin gut geeignet sind Cellulosederivate wie Methylcellulose und Carboxymethylcellulose. Besonders gut geeignet ist Poly-N-vinylpyrrolidon-(2).

Ganz besonders bevorzugt sind einpolymerisierbare, nichtionische Tenside vom Typ der Mono-(meth)acrylsäureester der Polyalkylenoxyde, wobei das OH-terminierte Molekülende auch z.B. mit Methyl endverschlossen vorliegen kann. Als Beispiele seien folgende Produkte von Nippon Oil & Fats genannt:

Blemmer 55 PET-800 :

$H_2C{=}CCH_3\text{-}CO\text{-}O\text{-}(EO/THF)_n\text{-}H$ mit M: 700-880 g/mol,

Blemmer 50 PMEP-800 B:

$H_2C{=}CCH_3\text{-}CO\text{-}O\text{-}(PO)_m(EO)_n\text{-}CH_3$ mit M: 800-900 g/mol wobei EO für Ethylenoxid, THF für Tetrahydrofuran und PO für Propylenoxid steht.

Im allgemeinen werden 0 bis 10 Gew.-Teile, bevorzugt 0,1 bis 5 Gew.-Teile und besonders bevorzugt 0,3 bis 2 Gew.Teile an Dispergatoren eingesetzt.

Die Adhäsivkomponenten gemäß dieser Erfindung können weiterhin bis zu 10 Gew.-Teilen üblicher Zusätze wie Stabilisatoren, Inhibitoren, Lichtschutzmittel, Farbstoffe, Pigmente oder Fluoreszenzstoffe enthalten.

Die erfindungsgemäßen Zubereitungen können hergestellt werden, indem man alle Komponenten a) bis d) bzw. a) bis e) durch kräftiges Rühren mischt.

Die erfindungsgemäßen Zubereitungen können als Adhäsivkomponente zur gleichzeitigen Behandlung von Zahnschmelz und Dentin verwendet werden. Die Behandlung des Dentins alleine wird dabei allerdings ebenfalls gegenüber der DE-OS 38 28 169 vereinfacht, da der dort notwendige Arbeitsschritt des Aufbringens eines Verschlußmaterials (vergl. Beispiel 12 der DE-OS 38 28 169) gemäß vorliegender Erfindung eingespart werden kann.

In einer besonderen Ausführungsform konditioniert man Zahnschmelz und Zahnbein gemeinsam vor der Behandlung mit der erfindungsgemäßen Zubereitung mit einer Flüssigkeit mit einem pH-Wert im Bereich von 0,1 bis 3,5.

Diese enthält im allgemeinen Säuren mit einem $pK_s$-Wert kleiner als 5 und gegebenenfalls eine amphotere Aminoverbindung mit einem $pK_s$-Wert im Bereich von 9,0 bis 10,6 und einem $pK_s$-Wert im Bereich von 11,5 bis 12,5. Folgende Säuren können z.B. in der Konditionierflüssigkeit enthalten sein:

Phosphorsäure, Salpetersäure, Brenztraubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel

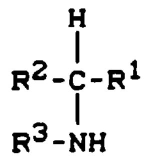

in der

$R^1$ für eine Carboxylgruppe steht,

$R^2$ Wasserstoff, gegebenenfalls durch Hydroxy, Thio, Methylthio, Carboxy, Amino, Phenyl, Hydroxy-phenyl oder die Gruppen

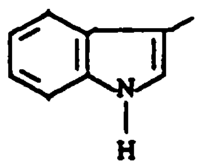 , 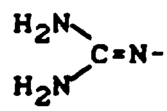 ,

substituierter Niederalkylrest,

$R^3$ Wasserstoff oder Phenyl bedeutet und wobei die Reste $R^1$ und $R^3$ durch einen Propylrest verbunden sein können, oder
in der

$R^1$ für Wasserstoff steht,

$R^2$ für die Gruppe

$$-A-NH_3X ,$$

in der

A für einen zweibindigen Alkylenrest mit 1 bis 6 Kohlenstoffatomen und

X für Halogen steht,
bedeutet und

$R^3$ Wasserstoff bedeutet,

genannt.

Beispielsweise seien diefolgenden amphoteren Aminoverbindungen genannt: Glycin, Serin, Threonin, Cystein, Tyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden solange freie Säurefunktionen verbleiben.

Konditionierflüssigkeiten, die mindestens eine der Säuren aus der Gruppe der Oxalsäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:

Bei einer Zahnreparatur trägt man beispielsweise nach einer mechanischen Präparation des Zahnschmelzes und Dentins zuerst die Konditionierflüssigkeit auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem kleinen Pinsel in einer dünnen Schicht auf und trocknet im Luftstrom. Nach der erfindungsgemäßen Behandlung trägt man die eigentliche Füllmasse, beispiels-weise im Dentalbereich übliche Kunststoffüllungsmassen (K. Eichner, "Zahnärztliche Werkstoffe und ihre Verarbeitung", Bd. 2, S. 135 ff, Hüthig Verlag, 5. Aufl. 1985) auf.

In ähnlicher Weise können die erfindungsgemäßen Zubereitungen zur Befestigung von Kronen, Brücken und ähnlichen Hilfsmitteln verwendet werden.

<u>Beispiele 1 bis 3</u> (Herstellung)

Die erfindungsgemäßen Adhäsivkomponenten und die vergleichskomponenten werden durch intensives Vermischen der in folgenden Beispielen aufgeführten Bestandteile erzeugt.

<u>Beispiel 1:</u>

| | |
|---|---|
| 23,4 g | N-Methacryloyloxyethyl-N-methylformamid |
| 5,6 g | Bis-GMA |
| 2,8 g | Essigsäure |
| 36,0 g | Wasser |
| 32,0 g | Ethanol |
| 0,06 g | Campherchinon |
| 0,14 g | p-N,N-Dimethylaminobenzolsulfonsäure-N',N'-diallylamid |

<u>Beispiel 2:</u>

| | |
|---|---|
| 23,3 g | N-Methacryloyloxyethyl-N-methylformamid |
| 5,6 g | Bis-GMA |
| 2,8 g | Essigsäure |
| 35,8 g | Wasser |
| 31,9 g | Ethanol |
| 0,06 g | Campherchinon |
| 0,14 g | p-N,N-Dimethylaminobenzolsulfonsäure-N',N'-diallylamid |
| 0,39 g | Blemmer PMEP-800 B |

<u>Beispiel 3</u>

| | |
|---|---|
| 26,6 g | N-Methacryloyloxyethyl-N-methylformamid |
| 5,6 g | Bis-GMA |
| 2,8 g | Essigsäure |
| 34,3 g | Wasser |
| 30,5 g | Ethanol |
| O,06 g | Campherchinon |
| 0,16 g | p-N,N-Dimethylaminobenzolsulfonsäure-N',N'-diallylamid |

Beispiele 4 und 5 (Vergleichsbeispiele)

Beispiel 4: (entsprechend Beispiel 8 der DE-A 38 28 169)

| | |
|---|---|
| 260 g | Wasser |
| 110 g | N-Methacryloyloxyethyl-N-methylformamid |
| 300 g | Campherchinon |

Beispiel 5:

| | |
|---|---|
| 24,1 g | N-Methacryloyloxyethyl-N-methylformamid |
| 5,8 g | Bis-GMA |
| 37,0 g | Wasser |
| 32,9 g | Ethanol |
| 0,06 g | Campherchinon |
| 0,14 g | p-N,N-Dimethylaminobenzolsulfonsäure-N',N'-diallylamid |

Die für die anwendungstechnischen Untersuchungen verwendete Konditionierlösung wird durch Vermischen folgender Bestandteile erhalten:

| | |
|---|---|
| 2,9 g | Glycin |
| 2,6 g | $Al(NO_3)_3$ x 9 $H_2O$ |
| 1,6 g | Oxalsäure x 2 $H_2O$ |
| 93,1 g | Wasser. |

Beispiel 6: (Anwendungstest, Bindungsfestigkeit)

Die Wirksamkeit und Eignung der Adhäsive (Beispiele 1 bis 5) wird überprüft durch Bestimmung der Scherbindungsfestigkeit auf Dentin und Schmelz. Es werden menschliche Zähne benutzt, die für max. drei Monate nach der Extraktion in 1 X Chloraminlösung aufbewahrt waren. Vor der Verwendung im Test werden die Zähne nach sorgfältiger Reinigung unter fließendem Wasser für mindestens drei und höchstens zehn Tage in physiologischer Kochsalzlösung gelagert. Am Tage vor der Verwendung im Bindungstest werden die Zähne einzeln mit Epoxidharz (®LEKUTHERM X20, Härter T3) in zylindrische Gummiformen von 25 mm Durchmesser und 12 mm Höhe eingebettet. Die Zähne werden durch Naßschleifen auf SiC-Papieren der Körnungen 240, 320, 400 und schließlich 600 soweit beschliffen, daß eine ausreichend große Schmelzoberfläche oder eine schmelznahe Dentinfläche zur Anbindung eines Kunststoffzylinders mit 3,5 mm Durchmesser freiliegt. Nach Abspülen mit entionisiertem Wasser und Trocknung im Luftstrom wird 30 Sekunden lang mit der Konditionierlösung und einem Wattepellet gereinigt, mit Wasser abgespült und getrocknet, bevor das Adhäsiv mit einem Pinsel aufgetragen, 30 Sekunden auf der Oberfläche belassen und dann im Druckluftstrom vorsichtig getrocknet wird. Die so vorbehandelte Probe wird in einer Einspannvorrichtung unter einer teilbaren Teflonform mit einer zylindrischen 3,5 mm weiten und 1 mm hohen Aufnahme festgeklemmt. Danach wird das Kunststoff-Füllungsmaterial ®Pekafill (U) mit einer Spritze in die zylindrische Form eingefüllt, mit einem $O_2$-undurchlässigen Strip abgedeckt und mit der Polymerisationsleuchte ®Translux CL (Kulzer) unter der aufliegenden Lichtaustrittsöffnung 60 Sekunden lang aktiviert. Unmittelbar im Anschluß daran wird die Probe aus der Halterung entfernt. Die Teflonform wird abgenommen und die Probe wird für 15 Minuten in 23°C warmen Wasser gelagert bis zur Einleitung der Scherbelastung, die mit Hilfe eines Druckstempels parallel zu und dicht an der Oberfläche des eingebetteten Zahnes unter einer Vorschubgeschwindigkeit von 1 mm/Minute bis zur Abtrennung erfolgt. Die Scherbindungsfestigkeit, der Quotient aus Bruchkraft und Kontaktareal am Zahn, wird jeweils an 5 Proben bestimmt und als deren Mittelwert und Standardabweichungen angegeben.

Beispiel 7 (Anwendungstest, Zahnkavität)

Zur Simulation der klinischen Anwendung von Adhäsiven und Kunststofffüllungsmaterialien werden in extrahierten Zähnen mit einer Vorgeschichte wie in Beispiel 6 Kavitäten präpariert und gefüllt. Als Maß für die Wirksamkeit wird die Adaption des Füllungsmaterials am Kavitätenrand bestimmt.

Die extrahierten Zähne werden auf einer unbeschädigten Aproximalseite auf SiC-Papier der Körnungen 240, 320, 400 und 600 naß beschliffen bis eine ausreichend große Dentinfläche zur Aufnahme einer ca. 3 mm weiten zylindrischen Kavität freiliegt. Die Kavität wird mit üblichen, zahnärztlichen Präparationsdiamanten mittlerer Körnung unter reichlich Wasserkühlung bis zu einer Tiefe von ca. 1,5 mm präpariert, dann mit Wasser abgespült und getrocknet. Mit einem getränkten Wattepellet wird wie im vorhergegangenen Beispiel 30 Sekunden lang die Kavität gereinigt, dann ausgewaschen und getrocknet bevor das Adhäsiv aufgepinselt, für 30 Sekunden belassen und schließlich getrocknet wird. Anschließend wird das Kunststoff-Füllungsmaterial ®Pekafill (U) mit einer Spritze in die Kavität eingefüllt. Der Überschuß wird mit einem $O_2$-undurchlässigen Strip vor Aktivierung (60 Sekunden) mit dem Lichtpolymerisationsgerät ®Translux CL (Kulzer) abgedeckt. Unmittelbar nach der Polymerisation wird der gefüllte Zahn für 15 Minuten in 23°C warmen Wasser aufbewahrt. Im Anschluß daran wird der Überschuß durch Schleifen auf feuchtem SiC-Papier der Körnungen 400 und 600 entfernt. Dabei werden ca. O,1 mm von der Kavitätenhöhe abgetragen. Der mit Wasser abgespülte Zahn wird im Luftstrom getrocknet und sofort im Auflichtmikroskop bei 500-facher Vergrößerung inspiziert. Die maximale Breite eines etwa vorhandenen Randspaltes wird mit Hilfe eines Schraubenokularmikrometers vermessen. Die mittlere, maximale Spaltbreite von je 5 Füllungen wird als Meßwert angegeben. Die mikroskopische Untersuchung des einzelnen Zahnes war in allen Fällen in weniger als 10 Minuten abgeschlossen. Damit wurde sichergestellt, daß die gemessenen Randspalten nicht durch Dehydrierung des Dentins entstanden oder in ihrer Breite beeinflußt waren.

Die Ergebnisse sind in der folgenden Tabelle zusammengefaßt:

| Zubereitung nach Beispiel Nr. | Scherbindungsfestigkeit [N/mm²] auf Schmelz | auf Dentin |
|---|---|---|
| 1 | 9.6+/-1.1 | 7.2+/-1.1 |
| 5*) | 8.7+/-1.2 | 5.6+/-1.1 |
| 2 | 14.4+/-3.3 | 8.9+/-0.1 |
| 3 | 10.7+/-1.6 | 9.9+/-2.1 |
| 4**) | < 2 | < 2 |

*) Vergleichsbeispiel: Zubereitung wie in Beispiel 1, jedoch ohne Essigsäure.

**) Vergleichsbeispiel gemäß DE-OS 38 28 169 unter Einhaltung der hier unter Beispiel 6 und 7 beschriebenen, vereinfachten Arbeitsweise.

Bei der Zubereitung gemäß Beispiel 1 wurde eine 100 %ige Randspaltfreiheit festgestellt, wohingegen bei der Zubereitung gemäß Vergleichsbeispiel 5 in 40 % der Fälle ein Randspalt auftritt.

## Patentansprüche

1. Zubereitungen enthaltend

a) Formamidgruppen enthaltende (Meth)acrylsäureester der Formel

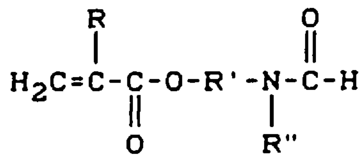

(1)

in der

R Wasserstoff oder Methyl bedeutet,

R' ein zweiwertiger aliphatischer ($C_2$ bis $C_5$) Rest bedeutet und

R" Wasserstoff oder ein einwertiger Alkylrest ($C_1$ bis $C_3$) bedeutet,

b) (Meth)acrylsaureester, die Vernetzungen ausbilden können,

c) Lösungsmittel,

d) gegebenenfalls Initiatoren, Coaktivatoren und Füllstoffe,
dadurch gekennzeichnet, daß

e) Säuren und gegebenenfalls

f) Dispergatoren

zugesetzt werden.

**2.** Zubereitungen gemäß Anspruch 1, dadurch gekennzeichnet, daß als Komponente (a) N-Methacryloyloxyethyl-N-methylformamid verwendet wird.

**3.** Zubereitungen gemäß Anspruch 1, dadurch gekennzeichnet, daß man von den Komponenten

(a) 2-80 Gew.-Teile,
(b) 0,3-80 Gew.-Teile,
(c) 10-1000 Gew.-Teile,
(d) Initiatoren 0-2 Gew.-Teile und Coaktivatoren 0-4 Gew.-Tle, jeweils bezogen auf die Summe der Komponenten (a) und (b), Füllstoffe 0-80 Gew.-Teile,
(e) 0,3-20 Gew.-Teile,
(f) 0-10 Gew.-Teile

zu deren Herstellung einsetzt, wobei sich die Mengenangaben zu den Komponenten, wenn nicht anders angegeben, auf die Gesamtzubereitung beziehen.

**4.** Zubereitungen gemäß Anspruch 1, dadurch gekennzeichnet, daß man von den Komponenten

(a) 5-50 Gew.-Teile,
(b) 1-50 Gew.-Teile,
(c) 30-300 Gew.-Teile,
(e) 1-15 Gew.-Teile

zu deren Herstellung einsetzt, wobei sich die Mengenangaben zu den Komponenten auf die Gesamtzubereitung beziehen.

**5.** Zubereitungen gemäß Anspruch 1, dadurch gekennzeichnet, daß man von den Komponenten

(a) 14-25 Gew.-Teile,
(b) 4-30 Gew.-Teile,

(c) 50-80 Gew.-Teile,
(e) 2-7 Gew.-Teile

zu deren Herstellung einsetzt, wobei sich die Mengenangaben zu den Komponenten auf die Gesamtzubereitung beziehen.

**6.** Zubereitungen gemäß Anspruch 1, dadurch gekennzeichnet, daß man als Komponente

(a) 23.4 Gew.-Teile N-Methacryloyloxyehtyl-N-methylformamid
(b) 5,6 Gew.-Teile Bis-GMA
(c) 36 Gew.-Teile Wasser und 32 Gew.-Teile Ethanol
(d) 0,06 Gew.-Teile Campherchinon und 0,14 Gew.-Teile p-N,N-Dimethylaminobenzolsulfonsäure-N',N'-diallylamid
(e) 2,8 Gew.-Teile Essigsäure

zu deren Herstellung einsetzt, wobei sich die Mengenangaben zu den Komponenten auf die Gesamtzubereitung beziehen.

**7.** Verwendung von Zubereitungen gemäß Anspruch 1 bei der Behandlung von Zahnschäden.

**8.** Verwendung von Zubereitungen gemäß Anspruch 1 bei der Herstellung von Dentaladhäsiven.

## Claims

**1.** Formulations containing

a) (meth)acrylic acid esters containing formamide groups, of the formula

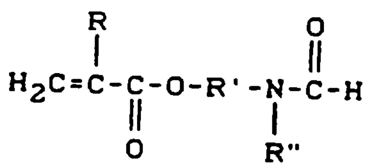

(1)

in which

R denotes hydrogen or methyl,

R' denotes a divalent aliphatic ($C_2$ to $C_5$) radical and

R" denotes hydrogen or a monovalent alkyl radical ($C_1$ to $C_3$),

b) (meth)acrylic acid esters which can form cross-linkings,

c) solvents, and

d) if appropriate initiators, coactivators and fillers,
characterised in that

e) acids
and if appropriate

f) dispersing agents
are added.

**2.** Formulations according to Claim 1, characterised in that N-methacryloyloxyethyl-N-methylformamide is used as component (a).

**3.** Formulations according to Claim 1, characterised in that of the components

(a) 2-80 parts by weight,
(b) 0.3-80 parts by weight,
(c) 10-1000 parts by weight,
(d) initiators 0.2 parts by weight and coactivators 0-4 parts by weight, in each case based on the sum of components (a) and (b), and fillers 0-80 parts by weight,
(e) 0.3-20 parts by weight and
(f) 0-10 parts by weight

are used for their preparation, the amounts stated for the components relating to the total formulation, unless stated otherwise.

**4.** Formulations according to Claim 1, characterised in that of the components

(a) 5-50 parts by weight,
(b) 1-50 parts by weight,
(c) 30-300 parts by weight and
(e) 1-15 parts by weight

are employed for their preparation, the amounts stated for the components being based on the total formulation.

**5.** Formulations according to Claim 1, characterised in that of the components

(a) 14-25 parts by weight,
(b) 4-30 parts by weight,
(c) 50-80 parts by weight and
(e) 2-7 parts by weight

are employed for their preparation, the amounts stated for the components being based on the total formulation.

**6.** Formulations according to Claim 1, characterised in that as component

(a) 23.4 parts by weight of N-methacryloyloxyethyl-N-methylformamide,
(b) 5.6 parts by weight of bis-GMA,
(c) 36 parts by weight of water and 32 parts by weight of ethanol,
(d) 0.06 parts by weight of camphorquinone and 0.14 parts by weight of p-N,N-dimethylaminobenzenesulphonic acid N',N'-diallylamide and
(e) 2.8 parts by weight of acetic acid

are employed for their preparation, the amounts stated for the components being based on the total formulation.

**7.** Use of formulations according to Claim 1 in the treatment of tooth damage.

**8.** Use of formulations according to Claim 1 in the preparation of dental adhesives.

**Revendications**

**1.** Préparations contenant

a) des esters d'acide (méth)acrylique contenant des groupes formamido, de formule

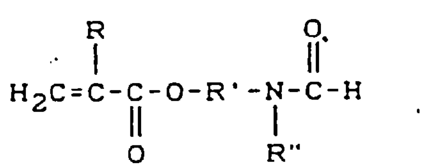 (1)

dans laquelle

R est de l'hydrogène ou un groupe méthyle,
R' est un reste aliphatique (en $C_2$ à $C_5$) divalent et
R" est de l'hydrogène ou un reste alkyle monovalent (en $C_1$ à $C_3$),

b) des esters d'acide (méth)acrylique qui peuvent créer des réticulations,
c) des solvants,
d) le cas échéant, des initiateurs, des co-activateurs et des charges,
caractérisées en ce que
e) des acides
et le cas échéant
f) des agents dispersants

sont ajoutés.

**2.** Préparations suivant la revendication 1, caractérisées en ce qu'on utilise comme composant (a) le N-méthacryloyloxyéthyl-N-méthylformamide.

**3.** Préparations suivant la revendication 1, caractérisées en ce qu'on utilise, pour les obtenir, des quantités de composants

(a) de 2 à 80 parties en poids,
(b) de 0,3 à 80 parties en poids,
(c) de 10 à 1000 parties en poids,
(d) de 0 à 2 parties en poids d'initiateurs et de 0 à 4 parties en poids de co-activateurs, dans chaque cas par rapport à la somme des composants (a) et (b), 0 à 80 parties en poids de charges,
(e) 0,3 à 20 parties en poids,
(f) 0 à 10 parties en poids,

les quantités indiquées de composants, sauf spécification contraire, se rapportant à la préparation totale.

**4.** Préparations suivant la revendication 1, caractérisées en ce qu'on utilise, pour les obtenir, des proportions de composants

(a) de 5 à 50 parties en poids,
(b) de 1 à 50 parties en poids,
(d) de 30 à 300 parties en poids,
(e) de 1 à 15 parties en poids,

les indications de quantités des composants se rapportant à la préparation totale.

**5.** Préparations suivant la revendication 1, caractérisées en ce qu'on utilise, pour les obtenir, des quantités des composants

(a) de 14 à 25 parties en poids,
(b) de 4 à 30 parties en poids,
(c) de 50 à 80 parties en poids,
(e) de 2 à 7 parties en poids,

les indications de quantités des composants se rapportant à la préparation totale.

**6.** Préparations suivant la revendication 1, caractérisées en ce qu'on utilise comme composants, pour les obtenir

(a) 23,4 parties en poids de N-méthacryloyloxyéthyl-N-méthylformamide
(b) 5,6 parties en poids de bis-GMA
(c) 36 parties en poids d'eau et 32 parties en poids d'éthanol

(d) 0,06 partie en poids de camphoquinone et 0,14 partie en poids de N',N'-diallylamide d'acide p-N, N-diméthylaminobenzènesulfonique
(e) 2,8 parties en poids d'acide acétique

les indications de quantités des composants se rapportant à la préparation totale.

**7.** Utilisation de préparations suivant la revendication 1 dans le traitement de lésions dentaires.

**8.** Utilisation de préparations suivant la revendication 1 dans la production d'adhésifs dentaires.